Europäisches Patentamt

European Patent Office

Office européen des brevets

(19)

(11) **EP 0 646 641 B1**

(12) # EUROPEAN PATENT SPECIFICATION

(45) Date of publication and mention
of the grant of the patent:
**03.03.1999 Bulletin 1999/09**

(51) Int Cl.6: **C11D 3/00**, C11D 3/386,
C11D 3/28

(21) Application number: **94307195.1**

(22) Date of filing: **30.09.1994**

(54) **Method of stabilizing preparation for contact lenses**

Verfahren zur Stabilisierung eines Kontaktlinsenpräparats

Méthode de stabilisation d'une préparation pour les lentilles de contact

(84) Designated Contracting States:
**AT BE CH DE DK ES FR GB GR IE IT LI LU MC NL PT SE**

(30) Priority: **01.10.1993 JP 270045/93**
**11.08.1994 JP 211834/94**

(43) Date of publication of application:
**05.04.1995 Bulletin 1995/14**

(73) Proprietor: **Senju Pharmaceutical Co., Ltd.**
**Osaka-shi, Osaka 541 (JP)**

(72) Inventors:
• **Nakayama, Hisayuki,**
**Lions Mansion Asagirigaoka**
**Akashi, Hyogo 673 (JP)**
• **Tsuchino, Kuniko**
**Kashiwara, Osaka 582 (JP)**
• **Kimoto, Akihiro**
**Kobe-shi, Hyogo 651-21 (JP)**

(74) Representative: **Burford, Anthony Frederick**
**W.H. Beck, Greener & Co.**
**7 Stone Buildings**
**Lincoln's Inn**
**London WC2A 3SZ (GB)**

(56) References cited:
WO-A-91/07192          DE-A- 4 038 692
GB-A- 1 354 761

• **DATABASE WPI Week 9333, Derwent Publications Ltd., London, GB; AN 93-261944 & JP-A-5 179 291 (LION CORP) 20 July 1993**

Remarks:
The file contains technical information submitted after the application was filed and not included in this specification

## Description

This invention is concerned with a method of stabilizing preparations for contact lenses containing proteolytic enzymes but not containing free calcium ions, which method comprises adding to the preparations a pyrrolidone compound selected from pyrrolidonecarboxylic acid and salts and esters thereof at a concentration of not less than 5 (w/v) % (as calculated when the preparation contains, or is dissolved in water to contain, 10 to 5,000 units/cm$^3$ of the proteolytic enzyme).

Contact lenses are roughly classified into two types; hard and soft contact lenses. Although hard contact lenses, with their non-hydrophilic property, usually show reduced oxygen permeability, hard contact lenses with improved oxygen permeability have recently been developed. However, both kinds of contact lenses are readily deposited with, for example, proteins, and in addition, oxygen-permeable lenses require daily care by cleaning (including cleansing), sterilization and preservation in order to maintain their desired oxygen permeability.

Proteolytic enzymes are used to remove protein adhered on the surface of contact lenses and there have been proposed and put in use a great variety of cleaning preparations containing proteolytic enzymes. For example, preparations composed mainly of proteolytic enzymes, which are supplied in the form of solids, such as tablets, granules and powders, are dissolved in, for example, purified water at the time of cleaning of contact lenses by their wearers. As a result, the wearers are forced to endure not only inconvenience from increased costs and troublesome procedures, but also they are troubled with time-course reduction in enzymatic activity after dissolution.

There have been proposed methods of stabilizing a proteolytic enzyme in solution; for example, JP-A-159822/1988 and JP-A-180515/1989 propose stabilizing a proteolytic enzyme by incorporating the proteolytic enzyme into a solution containing a water-miscible polyhydric alcohol. However, the resultant solution hardly exhibits enzymatic activity and, whilst it can be diluted with water to produce increased enzymatic activity, it suffers from the disadvantage of poor stability.

JP-A-5179291 discloses that the protease activity in a liquid detergent composition comprising a surfactant and a metal blocking agent (eg ethane-1,1-diphosphonate) is stabilized by free calcium ion and either or both alkanolamine and pyrrolidone carboxylate. It is stated that concentrations of pyrrolidone carboxylate in excess of 5 % by weight do not produce a marked increase in stability.

GB-A-1354761 discloses that protease and/or amylase activity in liquid enzyme preparations is stabilized by 1 to 45 % by weight of at least one water-soluble organic compound selected from:

(a) certain polyhydric aliphatic alcohols or ethers thereof;
(b) certain carboxylic or phosphonic acid amides or certain ureas;
(c) certain heterocyclic compounds; and
(d) certain dialkylsulphoxides,

and 1 to 30 % by weight of at least one water-soluble organic compound selected from:

(e) certain polyfunctional aliphatic or alicyclic amines;
(f) certain heterocyclic compounds; and
(g) certain carboxylic or phosphonic acid amides or certain ureas,

provided that (i) a compound of group (b) can only be used with a compound of group (e) or group (f) and (ii) a compound of group (c) can only be used with a compound of group (e) or group (g). Exemplified group (c) and group (f) heterocyclic compounds include 2-pyrrolidone.

It is an object of the present invention to stabilize a proteolytic enzyme in solution to provide a liquid preparation for contact lenses which, solely and without use of any auxiliaries, permits contact lenses to be simultaneously cleaned, sterilized and preserved.

The present inventors unexpectedly have found that addition of the pyrrolidone compound can lead to prolongation of the stability of proteolytic enzymes in a solution designed for use in cleaning contact lenses, without their activity deteriorating.

The present invention provides a method of stabilizing a liquid preparation not containing free calcium ions for contact lenses containing proteolytic enzymes which comprises adding the pyrrolidone compound to a solution containing an effective amount of the proteolytic enzyme, liquid preparations for contact lenses containing a proteolytic enzyme which is stabilized by use of the said method, and a process for producing such liquid preparations.

The pyrrolidone compounds which are selected in the present invention are D-, L- or DL- pyrrolidonecarboxylic acid (namely, 2-pyrrolidone-5-carboxylic acid) and their salts, for example, sodium salts, potassium salts or amine salts, such as triethanolamine salts, or their esters, for example, ethyl esters. The amount of such pyrrolidone compounds used suitably is chosen depending upon inter alia the kind of pyrrolidone compound, the type of contact lens to be cleaned, and the nature and extent of deposits to be removed. The presence of the pyrrolidone compound in the solution at a concentration of lower than 5 (w/v) % usually fails to provide satisfactory enzymatic stability. Consequently, such pyrrolidone compounds are used at a concentration of not less than 5 (w/v) %, preferably in the range of 10 to 60 (w/v) %.

The proteolytic enzymes which are useful in this invention include trypsin and chymotrypsin as well as proteases derived from microorganisms such as those of the genera Bacillus. The amount of such proteolytic enzymes is suitably determined based on the effective quantity sufficiently to achieve the intended cleaning effect, but preferably is 10 to 5,000 units/cm$^3$, more preferably 50 to 1,000 units/cm$^3$. When formulated in too small amounts proteolytic enzymes fail to produce a satisfactory cleaning effect and when used at too high concentrations incur the risk of causing damage to the skin during the cleaning procedure.

The preparations for contact lenses, which are prepared according to the present invention, desirably are adjusted to a pH value in the range of 4 to 8 for the purpose of stabilization of the proteolytic enzymes employed.

According to the present invention, the preparations for contact lenses can take the form of either solid or liquid, and the form of preparation is not limited provided that it is or can be formed into a solution on the occasion of use. In the case of liquid preparations, the solutions containing proteolytic-enzyme according to the present invention are especially useful and advantageous in that they not only develop enhanced enzymatic activity while keeping the proteolytic enzymes stable in aqueous solution, but also eliminate the need for dilution with water on the occasion of use as is normally the case with stabilized enzyme solutions for contact lenses containing polyhydric alcohols such as glycerol. As the solid form of preparation, there may be mentioned tablets, granules, powders and lyophilizates. Lyophilizates are preferred in that they dissolve quickly, show sterility and provide the composition with uniformity. The described amounts of the pyrrolidone compound, surfactant and proteolytic enzyme described herein are expressed in terms of those present in a solution prepared from the solid form on the occasion of use.

In addition to the proteolytic enzyme and the pyrrolidone compound, the preparation also can contain excipients, surfactants, preservatives, pH regulating agents, buffers, chelating agents, disintegrating agents and binders, as well as different enzymes, such as lipases, and other various additives.

As surfactants, nonionic, anionic and amphoteric surfactants are effective, and can be used in combination, if desired.

The anionic surfactants include, for example, sodium lauroylsarcosine, triethanolamine lauroyl-L-glutamate and sodium myristylsarcosine. Examples of amphoteric surfactants include lauryldimethylaminoacetic betaine, 2-alkyl-N-carboxymethyl-N-hydroxyethylimidazolinium betaine and alkyldiaminoglycine hydrochloride. As nonionic surfactants, there may be mentioned polysorbate 80, polyoxyethylenated hardened castor oil 60, polyoxyl 40 stearate and polyoxyethylene lauryl ether.

The amount of the surfactants to be formulated may arbitrarily be selected provided that the concentration achieves a satisfactory degree of enzymatic stability without causing any adverse effect on the contact lenses and ophthalmic tissues. Preferably, they are employed at concentrations in the range of 0.01 to 10 (w/v) %, more preferably 0.1 to 5 (w/v) %.

The preparations for contact lenses as stabilized according to the method of the present invention can be used by immersing a contact lens removed from the eyeball in 5 cm$^3$ of the preparation in the liquid form (e.g., the preparation for contact lenses in the aqueous solution state) for a period of not less than 30 min. to thereby accomplish spontaneous cleaning and sterilization simultaneously. The contact lens is then rinsed with tap water and replaced in the eye. A field test conducted by the present inventors indicated that the preparations for contact lenses in the solution state, after consecutive daily use for one week, caused no contraindication or problem.

The present invention can thus permit a proteolytic enzyme in a preparation for contact lenses to be maintained stable in the liquid state. Consequently, the stabilized preparation according to this invention can achieve simultaneous cleaning, sterilization and preservation of contact lenses, alone and without use of any additional means. It has the advantages that it can be used in a simple and convenient manner with deposits being effectively removed from contact lenses simply through soaking and standing and that no further treatment procedure such as dilution with water is required in the case of the solution preparation.

The experiment example and examples are described in the following to illustrate specifically the present invention, but it should be understood that these only illustrate this invention and do not limit its scope.

Experiment Example 1

Test on the removal of protein dirt:

Two oxygen-permeable hard contact lenses (made of siloxanyl methacrylate) adhered with artificially prepared dirt based on lysozyme chloride were soaked in cleaning solutions of the compositions as shown in Table 1 containing individually 10 % and 40 % of sodium DL-pyrrolidone- carboxylate and in a cleaning solution containing 40 % of glycerol, respectively, followed by standing for 15 hours. The contact lenses were washed with water and examined with the naked eye for removal of the artificial dirt deposited thereon. The results are tabulated below and confirm that the cleaning solutions with 10 % and 40 % sodium DL-pyrrolidonecarboxylate content removed the artificial dirt completely, whereas the cleaning solution containing 40 % of glycerol did not.

Results:

| Test solution | Before | After |
|---|---|---|
| 10 % of sodium DL-pyrrolidonecarboxylate | +++ | - |
| 40 % of sodium DL-pyrrolidonecarboxylate | +++ | - |
| 40 % of glycerol | +++ | +++ |
| Note: +++: a white turbid state observed on the lens surface.    - : no dirt observed on the lens surface. | | |

The contact lenses deposited with artificially prepared dirt were prepared by the following procedure:

The artificial dirt solution of the below-described formulation was prepared, degassed and heated at about 60°C. The lenses were placed in the solution until they became turbid to an appropriate degree, lumps of dirt removed, and then stored in water.

| Substance | Amount |
|---|---|
| Lysozyme chloride | 0.1 g |
| Disodium hydrogenphosphate | 0.2 g |
| Sodium hydroxide | q.s. |
| Purified water | q.s. |
| Total | 100 cm$^3$ (at pH 7.2) |

Example 1

The ingredients as described in Tables 1 and 2 were mixed to thereby prepare different test solutions. The thus-prepared test solutions were subjected to assay of enzymatic activity based on the Anson-Ogiwara's modified method immediately after being prepared and after being stored at a temperature of 30°C for 7 and 14 days, respectively. The residual rate of enzymatic activity (%) was calculated using the below-described equation, with the results being shown in Tables 1 and 2.

$$A \ (\%) = E/E_0 \text{ x } 100$$

where:

A =    Residual rate of enzymatic activity
E =    Enzymatic activity after being stored at 30°C for 7 or 14 days
$E_0$ =    Enzymatic activity immediately after being prepared.

As is shown in Tables 1 and 2, addition of the pyrrolidone compound to a solution containing a proteolytic enzyme was observed to result in marked stabilization of the proteolytic enzyme in the said solution.

Table 1:

| Formulation Substance | Reference B | B-1 | B-2 | B-3 | B-4 | B-5 | B-6 | B-7[*] |
|---|---|---|---|---|---|---|---|---|
| Bioprase (unit/cm$^3$) | 240 | 240 | 240 | 240 | 240 | 240 | 240 | 240 |
| Sodium DL-pyrrolidonecarboxylate | - | 60.0% | 40.0% | 30.0% | 20.0% | 10.0% | 5.0% | 0.5% |
| Sodium hydrogenphosphate | 0.2% | 0.2% | 0.2% | 0.2% | 0.2% | 0.2% | 0.2% | 0.2% |
| Phosphoric acid | q.s. | q.s. | q.s. | q.s. | q.s. | q.s. | q.s. | q.s. |
| Sodium hydroxide | q.s. | q.s. | q.s. | q.s. | q.s. | q.s. | q.s | q.s |
| Sodium chloride | 0.9% | 0.9% | 0.9% | 0.9% | 0.9% | 0.9% | 0.9% | 0.9% |
| Purified water | q.s. | q.s. | q.s. | q.s. | q.s. | q.s. | q.s. | q.s. |
| pH | 7.0 | 7.0 | 7.0 | 7.0 | 7.0 | 7.0 | 7.0 | 7.0 |
| Residual rate of enzymatic activity (%), at 30°C for 7 days | 69.5 | 99.2 | 99.1 | 87.9 | 94.5 | 84.5 | 84.8 | 70.2 |
| Residual rate of enzymatic activity (%), at 30°C for 14 days | 66.8 | 98.3 | 97.4 | 90.2 | 84.8 | 84.0 | 71.9 | 61.4 |

[*] outside the claimed range

EP 0 646 641 B1

EP 0 646 641 B1

Table 2:

| Formulation Substance | Reference T | T-1 | T-2 | T-3 | T-4 | T-5 |
|---|---|---|---|---|---|---|
| Proteolytic enzyme (unit/cm$^3$) | 240 | 240 | 240 | 240 | 240 | 240 |
| Sodium DL-pyrrolidonecarboxylate | – | 60.0% | 40.0% | 30.0% | 20.0% | 10.0% |
| Sodium hydrogenphosphate | 0.2% | 0.2% | 0.2% | 0.2% | 0.2% | 0.2% |
| Phosphoric acid | q.s. | q.s. | q.s. | q.s. | q.s. | q.s. |
| Sodium hydroxide | q.s. | q.s. | q.s. | q.s. | q.s. | q.s. |
| Sodium chloride | 0.9% | 0.9% | 0.9% | 0.9% | 0.9% | 0.9% |
| Purified water | q.s. | q.s. | q.s. | q.s. | q.s. | q.s. |
| pH | 7.0 | 7.0 | 7.0 | 7.0 | 7.0 | 7.0 |
| Residual rate of enzymatic activity (%), at 30°C for 7 days | 8.5 | 100.7 | 114.4 | 109.9 | 103.0 | 53.1 |
| Residual rate of enzymatic activity (%), at 30°C for 14 days | 4.8 | 70.8 | 89.6 | 66.5 | 78.5 | 36.7 |

Example 2

The ingredients as described in Table 3 were mixed to thereby prepare a test solution, which was assayed for enzymatic activity in the same manner as mentioned in Example 1, with the results being shown in Table 3.

As is evident from the test results shown in the columns B-6 of Table 1 and B-8 of Table 3, incorporation of the pyrrolidone compound as well as anionic surfactants and a nonionic surfactant was found to bring about enhanced stabilization of the proteolytic enzyme.

Example 3

| Substance | Amount |
|---|---|
| Bioprase | 1,500 units |
| Triethanolamine lauroyl-L-glutamate (30 %) | 0.1 g |
| Alkyldiaminoglycine hydrochloride | 0.03 g |
| Sodium DL-pyrrolidonecarboxylate (50 %) | 4.8 g |
| Boric acid | 0.03 g |
| Borax | 0.018 g |
| Sodium edetate | 0.006 g |
| Chlorhexidine gluconate | 0.3 mg |

The above-described ingredients were dissolved in 6 $cm^3$ of purified water to give a preparation for contact lenses containing the proteolytic enzyme, which was assayed for enzymatic activity in the same manner as described in Example 1. The preparation, after being stored at 30°C for 14 days, was found to retain not less than 95 % of the enzymatic activity and produced good cleaning effect. In the lipid- and protein-removal tests, the preparation was found to give satisfactory results, with no substantial microbial growth being noted.

Table 3:

| Formulation | |
|---|---|
| Substance | B-8 |
| Bioprase | 240 units/$cm^3$ |
| Sodium lauroylsarcosine [1] | 0.5% |
| Triethanolamine lauroyl-L-glutamate [1] | 0.5% |
| Sodium stearate[1] | 0.25% |
| Polysorbate 80[2] | 0.5% |
| Sodium DL-pyrrolidonecarboxylate | 5.0% |
| Disodium hydrogenphosphate | 0.2% |
| Phosphoric acid | q.s. |
| Sodium hydroxide | q.s. |
| Sodium chloride | 0.9% |
| Purified water | q.s. |
| pH | 7.0 |
| Residual rate of enzymatic activity (%), after storage for 14 days at 30°C. | 85.6 |

Notes: (1): Anionic surfactant
 (2) : Nonionic surfactant

Example 4

| Substance | Amount |
|---|---|
| Bioprase | 2,880 units |

(continued)

| Substance | Amount |
|---|---|
| Triethanolamine lauroyl-L-glutamate (30 %) | 0.2 g |
| Polyoxyl stearate 40 | 0.06 g |
| Lauryldimethylaminoacetate betaine (35 %) | 0.17 g |
| Boric acid | 0.03 g |
| Sodium edetate | 0.006 g |
| Sodium DL-pyrrolidonecarboxylate (50 %) | 3.6 g |

The above-described ingredients were dissolved in 6 cm$^3$ of purified water, followed by adjustment to pH 6.0 with hydrochloric acid to produce a preparation for contact lenses containing a proteolytic enzyme. The resultant test solution was subjected to the same test as described in Example 3, with similar, satisfactory results being obtained.

Example 5

| Substance | Amount |
|---|---|
| Bioprase | 2,800 units |
| Sodium lauroylsarcosinate | 0.06 g |
| Betaine lauryldimethylacetate (35 %) | 0.17 g |
| Polyoxyethylene lauryl ether | 0.03 g |
| Ethyl DL-pyrrolidonecarboxylate | 1.5 g |
| Sorbic acid | 0.006 g |
| Boric acid | 0.06 g |
| Borax | q.s. |

The above-described ingredients were dissolved in 6 cm$^3$ of purified water, followed by adjustment to pH 6.0 with hydrochloric acid to produce a preparation for contact lenses containing a proteolytic enzyme. The resultant test solution was subjected to the same test as described in Example 3, with similar, satisfactory results being obtained.

**Claims**

1.  A method of stabilizing a preparation not containing free calcium ions for contact lenses containing a proteolytic enzyme, characterized in that the said method comprises formulating the preparation with a pyrrolidone compound selected from pyrrolidonecarboxylic acid and salts and esters thereof at a concentration of not less than 5 (w/v) % (as calculated when the preparation contains, or is dissolved in water to contain, 10 to 5,000 units/cm$^3$ of the proteolytic enzyme).

2.  A method as claimed in Claim 1, wherein said concentration of pyrrolidone compound is 10 to 60 (w/v) %.

3.  A method as claimed in Claim 1 or Claim 2, wherein the preparation is liquid having a pH value in the range of 4 to 8 or solid which when dissolved in water has a pH value in said range.

4.  A method as claimed in any one of the preceding claims, wherein the proteolytic enzyme is trypsin, chymotrypsin or a protease originated from a microorganism belonging to the genus Bacillus.

5.  A method as claimed in any one of the preceding claims, wherein the preparation contains one or more nonionic, anionic or amphoteric surfactants.

6.  A method as claimed in Claim 5, wherein the surfactant concentration is in the range of 0.01 to 10 (w/v) %.

7.  A process for producing a preparation not containing free calcium ions for contact lenses, characterized in that said process comprises formulating the preparation with a proteolytic enzyme and pyrrolidone compound selected from pyrrolidonecarboxylic acid and salts and esters thereof at a concentration of not less than 5 (w/v) % (as calculated when the preparation contains, or is dissolved in water to contain, 10 to 5,000 units/cm$^3$ of the proteolytic enzyme).

8. A process as claimed in Claim 7, wherein said concentration of pyrrolidone compound is 10 to 60 (w/v) %.

9. A process as claimed in Claim 7 or Claim 8, wherein the preparation contains one or more nonionic, anionic or amphoteric surfactants.

10. A process as claimed in Claim 9, wherein the surfactant concentration is in the range of 0.01 to 10 (w/v) %.

11. A preparation not containing free calcium ions for contact lenses containing a proteolytic enzyme which comprises formulated therein a pyrrolidone compound selected from pyrrolidonecarboxylic acid and salts and esters thereof at a concentration of not less than 5 (w/v) % (as calculated when the preparation contains, or is dissolved in water to contain, 10 to 5,000 units/cm$^3$ of the proteolytic enzyme).

12. A preparation as claimed in Claim 11, wherein said concentration of pyrrolidone compound is 10 to 60 (w/v) %.

13. A preparation as claimed in Claim 11 or Claim 12, wherein the preparation contains one or more nonionic, anionic or amphoteric surfactants.

14. A preparation as claimed in Claim 13, wherein the surfactant concentration is in the range of 0.01 to 10 (w/v) %.

15. The use to stabilize a proteolytic enzyme in aqueous solution and in the absence of free calcium ions of a pyrrolidone compound selected from pyrrolidonecarboxylic acid and salts and esters thereof at a concentration of not less than 5 (w/v) % (as calculated when the preparation contains, or is dissolved in water to contain, 10 to 5,000 units/cm$^3$ of the proteolytic enzyme).

16. A use as claimed in Claim 15, wherein said concentration of pyrrolidone compound is 10 to 60 (w/v) %.

17. A use as claimed in Claim 15 or Claim 16, wherein the proteolytic enzyme is trypsin, chymotrypsin or a protease originated from a microorganism belonging to the genus Bacillus.

18. A method of cleaning contact lenses which comprises immersing a contact lens in an aqueous solution containing a proteolytic enzyme but not containing free calcium ions characterized in that said solution contains not less than 5 (w/v) % of a pyrrolidone compound selected from pyrrolidonecarboxylic acid and salts and esters thereof.

19. A method as claimed in Claim 18, wherein said concentration of pyrrolidone compound is 10 to 60 (w/v) %.

20. A method as claimed in Claim 18 or Claim 19, wherein the proteolytic enzyme is trypsin, chymotrypsin or a protease originated from a microorganism belonging to the genus Bacillus.

**Patentansprüche**

1. Verfahren zur Stabilisierung einer Zubereitung ohne einen Gehalt an freien Calciumionen für Kontaktlinsen, aber mit einem Gehalt an einem proteolytischen Enzym, dadurch gekennzeichnet, daß das genannte Verfahren das Formulieren der Zubereitung mit einer Pyrrolidonverbindung miteinschließt, die aus Pyrrolidoncarbonsäure sowie deren Salzen und Estern ausgewählt ist, wobei deren Konzentration nicht weniger als 5% (Gew./Vol.) beträgt (und zwar auf der Grundlage der Berechnung im Hinblick auf den Gehalt der Zubereitung an dem proteolytischen Enzym, wobei jene davon, ggf. erst nach Auflösen in Wasser, 10 bis 5.000 Einheiten/cm$^3$ enthält).

2. Verfahren nach Anspruch 1, bei dem die genannte Konzentration an Pyrrolidonverbindung 10 bis 60 % (Gew./Vol.) beträgt.

3. Verfahren nach Anspruch 1 oder 2, bei dem die Zubereitung als Flüssigkeit vorliegt und einen pH-Wert im Bereich von 4 bis 8 aufweist oder in fester Form vorliegt und nach dem Auflösen in Wasser einen pH-Wert in dem genannten Bereich aufweist.

4. Verfahren nach einem der vorstehenden Ansprüche, bei dem das proteolytische Enzym Trypsin, Chymotrypsin oder eine Protease darstellt, die von einem Mikroorganismus der Gattung Bacillus stammt.

5. Verfahren nach einem der vorstehenden Ansprüche, bei dem die Zubereitung ein oder mehrere nichtionische, anionische oder amphotere Tenside enthält.

6. Verfahren nach Anspruch 5, bei dem die Tensidkonzentration im Bereich von 0,01 bis 10 % (Gew./Vol.) liegt.

7. Verfahren zur Herstellung einer Zubereitung ohne Gehalt an freien Calciumionen für Kontaktlinsen, dadurch gekennzeichnet, daß das genannte Verfahren die Formulierung der Zubereitung mit einem proteolytischen Enzym und einer Pyrrolidonverbindung miteinschließt, die aus Pyrrolidoncarbonsäure sowie deren Salzen und Estern ausgewählt ist, wobei deren Konzentration nicht weniger als 5 % (Gew./Vol.) beträgt (und zwar auf der Grundlage der Berechnung im Hinblick auf den Gehalt der Zubereitung an dem proteolytischen Enzym, wobei jene, ggf. erst nach dem Auflösen in Wasser, 10 bis 5.000 Einheiten/cm$^3$ enthält).

8. Verfahren nach Anspruch 7, bei dem die genannte Konzentration der Pyrrolidonverbindung 10 bis 60 % (Gew./Vol.) beträgt.

9. Verfahren nach Anspruch 7 oder 8, bei dem die Zubereitung ein oder mehrere nichtionische, anionische oder amphotere Tenside enthält.

10. Verfahren nach Anspruch 9, bei dem die Tensidkonzentration im Bereich von 0,01 bis 10 % (Gew./Vol.) liegt.

11. Zubereitung ohne einen Gehalt an freien Calciumionen für Kontaktlinsen, aber mit einem Gehalt an einem proteolytischen Enzym, wobei die Zubereitung in der Formulierung eine Pyrrolidonverbindung miteinschließt, die aus Pyrrolidoncarbonsäure sowie deren Salzen und Estern ausgewählt ist, wobei deren Konzentration nicht weniger als 5 % (Gew./Vol.) beträgt (und zwar auf der Grundlage der Berechnung im Hinblick auf den Gehalt der Zubereitung an dem proteolytischen Enzym, wobei jene davon, ggf. erst nach dem Auflösen in Wasser, 10 bis 5.000 Einheiten/cm$^3$ enthält).

12. Zubereitung nach Anspruch 11, worin die genannte Konzentration an der Pyrrolidonverbindung 10 bis 60 % (Gew./Vol.) beträgt.

13. Zubereitung nach Anspruch 11 oder 12, worin ein oder mehrere nichtionische, anionische oder amphotere Tenside enthalten sind).

14. Zubereitung nach Anspruch 13, worin die Tensidkonzentration im Bereich von 0,01 bis 10 % (Gew./Vol.) liegt.

15. Verwendung einer Pyrrolidonverbindung,

die aus Pyrrolidoncarbonsäure sowie deren Salzen und Estern ausgewählt ist, in einer Konzentration von nicht weniger als 5 % (Gew./Vol.) (und zwar auf der Grundlage der Berechnung im Hinblick auf den Gehalt der Zubereitung an dem proteolytische Enzym, wobei jene davon, ggf. erst nach Auflösen in Wasser, 10 bis 5.000 Einheiten/cm$^3$ enthält),

zur Stabilisierung eines proteolytischen Enzyms in wäßriger Lösung und in Abwesenheit freier Calciumionen.

16. Verwendung nach Anspruch 15, bei der die Konzentration an der Pyrrolidonverbindung 10 bis 60 % (Gew./Vol.) beträgt.

17. Verwendung nach Anspruch 15 oder 16, bei der das proteolytische Enzym Trypsin, Chymotrypsin oder eine Protease darstellt, die aus einem zur Gattung Bacillus gehörenden Mikroorganismus stammt.

18. Verfahren zur Reinigung von Kontaktlinsen, welches das Eintauchen einer Kontaktlinse in eine wäßrige Lösung mit einem Gehalt an einem proteolytischen Enzym umfaßt, wobei die Lösung jedoch keine freien Calciumionen enthält, dadurch gekennzeichnet, daß die Lösung eine Pyrrolidonverbindung, die aus Pyrrolidoncarbonsäure sowie deren Salzen und Estern ausgewählt ist, in einer Konzentration von nicht weniger als 5% (Gew./Vol.) enthält.

**19.** Verfahren nach Anspruch 18, bei dem die genannte Konzentration an der Pyrrolidonverbindung 10 bis 60 % (Gew./Vol.) beträgt.

**20.** Verfahren nach Anspruch 18 oder 19, bei dem das proteolytische Enzym Trypsin, Chymotrypsin oder eine Protease darstellt, die aus einem zur Gattung Bacillus gehörenden Mikroorganismus stammt.

## Revendications

**1.** Procédé de stabilisation d'une préparation ne contenant pas d'ions calcium libres pour lentilles de contact contenant une enzyme protéolytique, caractérisé en ce que ledit procédé comprend la formulation de la préparation avec un composé de la pyrrolidone choisi parmi l'acide pyrrolidone-carboxylique et les sels et esters de celui-ci à une concentration ne valant pas moins de 5% (poids/volume) (telle que calculée quand la préparation contient, ou est dissoute dans de l'eau pour contenir, 10 à 5000 unités/cm$^3$ de l'enzyme protéolytique).

**2.** Procédé selon la revendication 1, dans lequel ladite concentration du composé de la pyrrolidone est comprise entre 10 et 60% (poids/volume).

**3.** Procédé selon la revendication 1 ou la revendication 2, dans lequel la préparation est un liquide ayant un pH d'une valeur comprise entre 4 et 8 ou un solide qui lorsqu'il est dissous dans l'eau a un pH d'une valeur comprise dans ledit intervalle.

**4.** Procédé selon l'une quelconque des revendications précédentes, dans lequel l'enzyme protéolytique est la trypsine, la chymotrypsine ou une protéase originaire d'un micro-organisme appartenant au genre Bacillus.

**5.** Procédé selon l'une quelconque des revendications précédentes, dans lequel la préparation contient un ou plusieurs tensioactifs non ioniques, anioniques ou amphotères.

**6.** Procédé selon la revendication 5, dans lequel la concentration de tensioactif est comprise entre 0,01 et 10% (poids/volume).

**7.** Procédé de production d'une préparation ne contenant pas d'ions calcium libres pour lentilles de contact, caractérisé en ce que ledit procédé comprend la formulation de la préparation avec une enzyme protéolytique et un composé de la pyrrolidone choisi parmi l'acide pyrrolidone-carboxylique et les sels et esters de celui-ci à une concentration ne valant pas moins de 5% (poids/volume) (telle que calculée quand la préparation contient, ou est dissoute dans de l'eau pour contenir, 10 à 5000 unités/cm$^3$ de l'enzyme protéolytique).

**8.** Procédé selon la revendication 7, dans lequel ladite concentration du composé de la pyrrolidone est comprise entre 10 et 60% (poids/volume).

**9.** Procédé selon la revendication 7 ou la revendication 8, dans lequel la préparation contient un ou plusieurs tensioactifs non ioniques, anioniques ou amphotères.

**10.** Procédé selon la revendication 9, dans lequel la concentration de tensioactif est comprise entre 0,01 et 10% (poids/volume).

**11.** Préparation ne contenant pas d'ions calcium libres pour lentilles de contact contenant une enzyme protéolytique, qui comprend, formulé dans celle-ci, un composé de la pyrrolidone choisi parmi l'acide pyrrolidone-carboxylique et les sels et esters de celui-ci à une concentration ne valant pas moins de 5% (poids/volume) (telle que calculée quand la préparation contient, ou est dissoute dans de l'eau pour contenir, 10 à 5000 unités/cm$^3$ de l'enzyme protéolytique).

**12.** Préparation selon la revendication 11, dans laquelle ladite concentration du composé de la pyrrolidone est comprise entre 10 et 60% (poids/volume).

**13.** Préparation selon la revendication 11 ou la revendication 12, dans laquelle la préparation contient un ou plusieurs tensioactifs non ioniques, anioniques ou amphotères.

14. Préparation selon la revendication 13, dans laquelle la concentration de tensioactif est comprise entre 0,01 et 10% (poids/volume).

15. Utilisation, pour stabiliser une enzyme protéolytique en solution aqueuse et en l'absence d'ions calcium libres, d'un composé de la pyrrolidone choisi parmi l'acide pyrrolidone-carboxylique et les sels et esters de celui-ci à une concentration ne valant pas moins de 5% (poids/volume) (telle que calculée quand la préparation contient, ou est dissoute dans de l'eau pour contenir, 10 à 5000 unités/cm$^3$ de l'enzyme protéolytique).

16. Utilisation selon la revendication 15, dans laquelle ladite concentration du composé de la pyrrolidone est comprise entre 10 et 60% (poids/volume).

17. Utilisation selon la revendication 15 ou la revendication 16, dans laquelle l'enzyme protéolytique est la trypsine, la chymotrypsine ou une protéase originaire d'un micro-organisme appartenant au genre Bacillus.

18. Procédé de nettoyage de lentilles de contact, qui comprend l'immersion d'une lentille de contact dans une solution aqueuse contenant une enzyme protéolytique mais ne contenant pas d'ions calcium libres, caractérisé en ce que ladite solution ne contient pas moins de 5% (poids/volume) d'un composé de la pyrrolidone choisi parmi l'acide pyrrolidone-carboxylique et les sels et esters de celui-ci.

19. Procédé selon la revendication 18, dans lequel ladite concentration du composé de la pyrrolidone est comprise entre 10 et 60% (poids/volume).

20. Procédé selon la revendication 18 ou la revendication 19, dans lequel l'enzyme protéolytique est la trypsine, la chymotrypsine ou une protéase originaire d'un micro-organisme appartenant au genre Bacillus.